(19) Europäisches Patentamt / European Patent Office / Office européen des brevets

(11) **EP 3 290 060 B1**

(12) **EUROPEAN PATENT SPECIFICATION**

(45) Date of publication and mention of the grant of the patent:
**26.02.2020 Bulletin 2020/09**

(51) Int Cl.:
***A61L 27/16*** *(2006.01)*     ***A61L 27/54*** *(2006.01)*

(21) Application number: **17188553.6**

(22) Date of filing: **30.08.2017**

(54) **ANTIBACTERIAL POLYMERIC MATERIAL AND ORTHOPAEDIC PROSTHETIC DEVICE OBTAINED THEREFROM**

ANTIBAKTERIELLES POLYMERISCHES MATERIAL UND EIN ORTHOPÄDISCHES GERÄT BESTEHEND DARAUS

MATÉRIAU POLYMÉRIQUE ANTIBACTÉRIEN ET PROTHÈSE ORTHOPÉDIQUE FAITE DE CE MATÉRIAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.08.2016 IT 201600088606**

(43) Date of publication of application:
**07.03.2018 Bulletin 2018/10**

(73) Proprietor: **Parx Plastics N.V.**
**3006 AC Rotterdam (NL)**

(72) Inventors:
• **VAN DER JAGT, Michael**
**3065NB ROTTERDAM (NL)**
• **FIORI, Michele**
**40129 BOLOGNA (IT)**
• **COSTA, Luigi**
**10153 TORINO (IT)**
• **ZANASI, Stefano**
**41014 CASTELVETRO DI MODENA (MODENA) (IT)**
• **MARCHETTI, Marco**
**63062 MONTEFIORE DELL'ASO (ASCOLI PICENO) (IT)**
• **VANDINI, Alberta**
**44011 ARGENTA (FERRARA) (IT)**

(74) Representative: **Rossetti, Elena**
**Bugnion S.p.A.**
**Viale Lancetti 17**
**20158 Milano (IT)**

(56) References cited:
**US-A1- 2011 112 646     US-A1- 2014 024 736**
**US-A1- 2014 296 442**

## Description

The present invention relates to an antibacterial polymeric material comprising ultrahigh molecular weight polyethylene (UHMWPE), vitamin E and at least one organic zinc compound and an implantable orthopedic prosthetic device having antibacterial properties obtained therefrom.

State of the art

Ultrahigh molecular weight polyethylene (UHMWPE) has been used for several decades as a component of implantable prosthetic devices for use in the orthopedic field.

This polymer, in fact, is characterized by high biocompatibility, low water absorption, an extremely low coefficient of friction and high abrasion resistance. Given its particular characteristics, UHMWPE is normally used to make the sliding surfaces of joint prostheses, in conjunction with metal parts, in particular in the manufacture of prostheses of the hip, knee, ankle, elbow and vertebral column.

Although the high abrasion resistance of UHMWPE ensures a decidedly long average lifespan of implants made with this material, even UHMWPE has a limited durability over time and may undergo phenomena of surface wear due to surface oxidation of the material, which leads to the formation of debris, tied to possible inflammatory phenomena.

In order to reduce the wear of UHMWPE, this material is subjected to irradiation with beta (β) or gamma (γ) rays, which promote the cross-linking of the polymer chains. However, this treatment causes the formation of free macroradicals, which may have a negative effect on the physical and mechanical properties of the material, thus reducing its durability and performance.

In order to counter the effect of free radicals, UHMWPE can be subjected to heat stabilization (annealing or remelting). However, heat treatments negatively impact the mechanical properties of the material. More recently, the market has seen the introduction of UHMWPEs containing vitamin E, an antioxidant added to the polymer so that it will react preferentially with the free radicals, thereby preserving the polymer itself, without altering the physical and mechanical properties of the material. Thanks also to the approval of standard ASTM F2695-07, UHMWPE containing vitamin E has rapidly become the standard material for manufacturing orthopedic prosthetic devices.

Despite the evolution of surgical techniques and prophylaxis, infections of joint prostheses (Periprosthetic Joint Infections, PJI) are among the complications most often associated with arthroplasty interventions.

These infections, due to various bacterial strains, may occur both a short time after the surgical intervention and as late infections, and they can constitute a serious problem for the patient, who will see a delay in the full functional recovery of the limb operated on.

In the case of both early postoperative infections and late infections, the most widely used treatment protocol provides for the prostheses to be explanted and cleaned, and antibiotic treatment of the patient, normally extended for at least two weeks, followed by subsequent re-implantation.

The average percentage of infection is about 1.5-2.0% for hip prostheses and 2.5-5.0 % for knee prostheses; in any case, it cannot be reduced to below 0.5%. Considering that the cost of revising a septic prosthesis can be up to five times greater than the cost of a first implant, it appears evident that the economic impact of prosthetic infections is extremely high (F. Ciucio et al., Bollettino of the società medico chirurgica of Pavia, 2013, 126(2): 421-430).

US 2014/024736 A1 discloses implantable medical devices including modified polymers. The polymers can have antioxidants (such as vitamin E) and/or biological agents (e.g. antimicrobials, such as zinc ions) bonded to the molecule. The polymer is preferably UHMWPE. The implant may be a prosthetic knee replacement system.

Scientific evidence has demonstrated that prosthetic infections are mainly due to the formation of a highly antibiotic-resistant biofilm on the surface of the implants. Most experts agree on the fact that the difference between early and delayed postoperative infections is closely linked to the entity and extent of the biofilm. Once a biofilm has formed, there are no alternatives to surgical revision of the prosthetic implant.

There is thus a strongly felt need to further reduce postoperative complications due to periprosthetic infections, in particular by acting on the formation of and/or reducing biofilms.

In this context, one object of the present invention is to provide a material that can be used for the manufacture of intrinsically antimicrobial prosthetic joint implants and which is capable of preventing, reducing and/or eliminating bacterial proliferation on the surface of the material itself.

A further object of the present invention is to provide a process for the preparation of said material.

A further object of the present invention is to provide an orthopedic prosthetic device comprising said material.

Summary of the invention

In a first aspect, the present invention relates to an antibacterial polymeric material comprising ultrahigh molecular weight polyethylene (UHMWPE), $\leq 0{,}3$ wt.% of vitamin E and $\leq 0.1$ wt.% of Zn, wherein the zinc is added to the ultrahigh molecular weight polyethylene in the form of at least one zinc compound selected among an inorganic zinc salt, a zinc carboxylate, zinc pyrithione, zinc acetylacetonate and mixtures thereof.

In a further aspect thereof, the present invention relates to a process for the preparation of the antibacterial polymeric material according to the invention comprising mixing UHMWPE with $\leq 0.3$ wt.% of vitamin E, and $\leq 0.1$ wt.% of Zn, in the form of at least one zinc compound as described above, wherein the mixing is carried out in the dry state, in solution or in the molten state.

In a further aspect, the present invention relates to the use of the antibacterial polymeric material as described above for the production of an orthopedic prosthetic device.

Therefore, in a further aspect, the present invention relates to an antibacterial orthopedic prosthetic device comprising the antibacterial polymeric material according to the invention.

Brief description of the figures

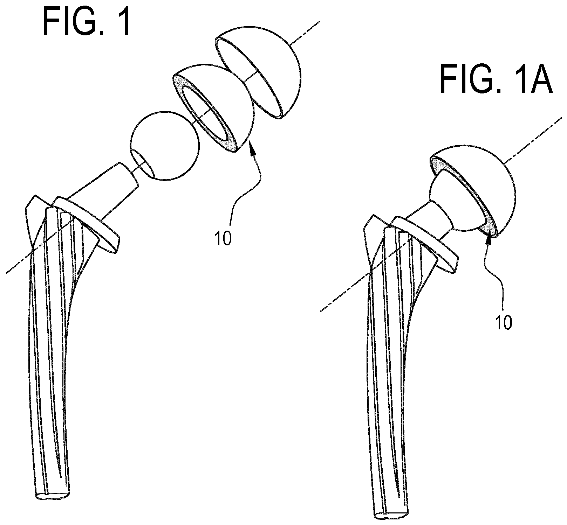

The invention will be illustrated below in detail, also with reference to Figures 1 and 1A, in which a hip endoprosthesis is shown.

Detailed description of the invention

In the present description and in the appended claims, the weight percentages of vitamin E and Zinc (expressed as an element) refer to the total weight of the antibacterial polymeric material.

In the present description and in the appended claims, the term "UHMWPE" refers to ultrahigh molecular weight polyethylene. The term "virgin UHMWPE" refers to ultrahigh molecular weight polyethylene that is not doped with vitamin E.

In the present description and in the appended claims, the term "antibacterial" refers to a substance, compound or material having bactericidal and/or bacteriostatic properties.

In the present invention, the term "orthopedic prosthetic device" refers to an endoprosthesis intended to be surgically implanted in the body of a human or animal to replace, in whole or in part, different skeletal components. An orthopedic prosthetic device can be a total joint endoprosthesis, which surgically replaces the whole joint (total endoprosthesis or arthroprosthesis) or else a partial joint endoprosthesis. In a first aspect, the present invention relates to an antibacterial polymeric material comprising ultrahigh molecular weight polyethylene (UHMWPE), vitamin E and $\leq 0.1$ wt.% of Zn, wherein the Zn is added to the UHMWPE in the form of at least one zinc compound selected among an inorganic zinc salt, a zinc carboxylate, zinc pyrithione, zinc acetylacetonate and mixtures thereof.

The ultrahigh molecular weight polyethylene (UHMWPE) usable for the implementation of the present invention can be a polyethylene homopolymer having a molecular weight preferably $\geq 2 \times 10^6$ amu, preferably comprised between $2 \times 10^6$ and $6 \times 10^6$ amu.

Furthermore, in order to be suitable for the production of a prosthetic joint device, the UHMWPE usable for the implementation of the present invention can have at least one of the following characteristics; preferably it can possess all of the following physical and mechanical characteristics:

- crystallinity about 40-65%; and/or
- density $\geq$ about 0.930 g/cm$^3$, preferably comprised between about 0.930 and about 0.945 g/cm$^3$; and/or
- ultimate tensile strength $\geq$ about 35 MPa; and/or
- yield strength $\geq$ about 20 MPa; and/or
- elongation at break $\geq$ about 300%.

According to a preferred variant, the virgin UHMWPE usable for the implementation of the present invention can satisfy the requirements established by standard ASTM F648.

The ultrahigh molecular weight polyethylene usable for the implementation of the present invention is generally produced using catalysts for ethylene polymerization, in particular of the Ziegler-Natta type, in processes known in the art, and is presently commercially available.

The antibacterial polymeric material according to the invention comprises $\leq 0.3$ wt.%, more preferably about 0.05 - 0.3 wt.%, even more preferably about 0.1 wt.% of vitamin E. The vitamin E consists of a mixture of tocopherols and tocotrienols, wherein $\alpha$-tocopherol is the most abundant chemical species.

The presence of vitamin E in the antibacterial polymeric material of the invention makes it possible to minimize the polymer degradation reactions due to the presence of the free radicals that form in the orthopedic prosthetic device as a result of irradiation. The concentration of vitamin E in the ranges specified above does not negatively impact the physical and mechanical characteristics of UHMWPE or of the orthopedic prosthetic devices obtained from it.

The antibacterial polymeric material of the invention can comprise about 0.01-0.1 wt.% of Zn, preferably about 0.03 - 0.09 wt.% of Zn.

The zinc can be added to the UHMWPE in the form of at least one inorganic zinc salt preferably selected among zinc dichloride, zinc sulfate, zinc carbonate, zinc basic carbonate, zinc nitrate, zinc phosphate and mixtures thereof, preferably zinc carbonate.

Alternatively, the at least one zinc carboxylate can be selected among the zinc compounds with carboxylic acids of formula $R^1C(=O)OH$ and/or with anions of said carboxylic acids, wherein $R^1$ can be selected among a C1-C17 alkyl, C1-C10 substituted alkyl, C1-C10 alkenyl, phenyl, substituted phenyl, heterophenyl and pyrrolidone.

The terms "alkyl" and "alkenyl" refer to straight or branched monovalent groups derived, respectively, from an alkane and an alkene by removal of a hydrogen atom.

The terms "substituted alkyl" and "substituted phenyl" refer, respectively, to an alkyl and phenyl group in which at least one hydrogen atom is substituted with at least one group selected among $-NH_2$, $-OH$, $=O$ and $- O^{(-)}$.

The term "heterophenyl" refers to a phenyl group in which at least one carbon atom is substituted by at least one heteroatom selected among N, O and S, preferably N.

The term "pyrrolidone" refers to a monovalent substituent derived from pyrrolidone by removal of a hydrogen atom.

The zinc compounds listed above can be zinc salts, covalent zinc compounds or zinc complexes with carboxylic acids.

Preferably, said at least one zinc carboxylate can be selected among zinc acetate, zinc propionate, zinc acrylate, zinc methacrylate, zinc oxalate, zinc aspartate, zinc citrate, zinc gluconate, zinc neodecanoate, zinc undecylenate, zinc stearate, Zn PCA (zinc salt of pyrrolidone carboxylic *acid*), zinc picolinate, zinc salicylate and mixtures thereof, preferably between zinc acetate and zinc stearate.

The antibacterial polymeric material according to the invention can be prepared by means of a process that comprises mixing UHMWPE with ≤ 0,3 wt.% of vitamin E, preferably with about 0.05 - 0.3 wt.%, even more preferably with about 0.1 wt.% of vitamin E, and ≤ 0.1 wt.% of Zn, preferably about 0.01-0.1 wt.%, more preferably about 0.03 - 0.09 wt.% of Zn, in the form of at least one Zn compound as described above, wherein the mixing is carried out in the dry state, in solution or in the molten state.

The UHMWPE can be mixed with vitamin E and at least one zinc compound as described above by adding said additives to the UHMWPE in any order whatsoever.

Preferably, the virgin UHMWPE can be premixed with vitamin E and the at least one Zn compound can subsequently be added as described above. This variant of the process can prove to be particularly advantageous, since there are commercially available UHMWPEs already doped with vitamin E, also in the preferred concentrations for the implementation of the present invention. The UHMWPE premixed with vitamin E usable in this variant of the process can preferably satisfy the requirements established by standard ASTM F2695-07.

According to a first embodiment, the process can comprise the following steps:

(i) providing a UHMWPE premixed with vitamin E, with ≤ 0.3 wt.%, preferably about 0.05 - 0.3 wt.%, even more preferably about 0.1 wt.% of vitamin E;
(ii) mixing, in solution or in the dry state, the UHMWPE premixed with vitamin E with ≤ 0.1 wt.% of Zn, preferably about 0.01-0.1 wt.%, more preferably about 0.03 - 0.09 wt.% of Zn, wherein the zinc is added to the UHMWPE in the form of at least one zinc compound as described above, so as to obtain an unconsolidated polymeric material;
(iii) consolidating the polymeric material.

According to this first embodiment, step (i) of the process can be carried out by mixing the vitamin E with the UHMWPE in the molten state. Advantageously, however, step (i) can be carried out using a commercially available UHMWPE doped with vitamin E as previously described, in the form of granules or powder, preferably powder.

In step (ii), the at least one zinc compound as described above can be added to the UHMWPE doped with vitamin E in solution. According to this variant step (ii) can comprise:

(ii.a) providing a solution comprising at least one zinc compound as described above and at least one solvent capable of solubilizing the at least one zinc compound, wherein the UHMWPE premixed with vitamin E is insoluble;
(ii.b) mixing the UHMWPE premixed with vitamin E with the solution comprising the at least one Zn compound;
(ii.c) evaporating said at least one solvent so as to obtain an unconsolidated antibacterial polymeric material.

The solvent used in step (ii.a) can be water, at least one water-miscible solvent and mixtures thereof, preferably water. The concentration and amount of the solution used in step (ii.b) are such as to obtain a concentration ≤ 0.1 wt.% of Zn, preferably about 0.01-0.1 wt.%, more preferably about 0.03 - 0.09 wt.% of Zn in the antibacterial polymeric material obtained from the process.

Step (ii.b) can be carried out under stirring at room temperature and ambient pressure. The person skilled in the art will be able, based on his technical knowledge, to determine the process conditions (stirring time and speed) in order to obtain a homogeneous dispersion of the zinc compound.

The temperature and pressure of step (ii.c) of evaporating the solvent depend on the at least one solvent used in the process. The evaporation temperature, at a given pressure, will have to be higher than the boiling temperature of the solvent. At the end of step (ii.c), a polymeric material is obtained, preferably in powder form, on which the at least one zinc compound is deposited. This first variant of the process for the production of the antibacterial polymeric material according to the invention is particularly advantageous since the zinc compound is uniformly distributed over the surface of the polymer, thus ensuring an optimal distribution of the zinc in the material during the consolidation step (iii).

Alternatively, in step (ii), the at least one zinc compound as described above can be mixed in the dry state with the UHMWPE doped with vitamin E. According to this variant, step (ii) can be carried out by mixing suitable amounts of a powder of at least one zinc compound as described above with a UHMWPE comprising vitamin E, preferably also in powder form, using a dry mixing apparatus known in the art, for example a turbo-mixer. This second variant of the process is less preferred, since the dispersion of the at least one zinc compound in the polymeric material is less uniform. At the end of step (ii), the polymeric material can be consolidated by means of compression molding or ram extrusion, preferably by ram extrusion (step iii).

Compression molding is a molding method in which the material, generally preheated, is placed in a heated open mold cavity. The mold is closed and pressure is applied to force the material into contact with all areas of the mold, while the heat and pressure are maintained until the consolidated material is obtained.

In the ram extrusion process, the material, generally in powder form, is fed to a chamber and a hydraulic piston (hydraulic ram) pushes the material from this chamber into the mold, where it is heated. The hydraulic piston moves continuously back and forth in order to feed the material to the mold and force the consolidated material through the extrusion head.

As the physical and mechanical characteristics of the antibacterial polymeric material according to the invention are substantially unchanged from those of the virgin UHMWPE present on the market, the industrial consolidation processes presently used in the manufacture of UHMWPE need not be substantially modified in order to obtain the antibacterial polymeric material of the invention and the person skilled in the art will be able to determine, based on his technical knowledge, the process conditions for consolidating the material.

In a further embodiment, the at least one zinc compound as described above can be added to the UHMWPE doped with vitamin E in the form of a concentrated masterbatch. According to this embodiment, the process for the production of an antibacterial polymeric material can comprise the following steps:

(I) providing a UHMWPE premixed with vitamin E, with ≤ 0.3 wt.%, preferably about 0.05 - 0.3 wt.%, even more preferably about 0.1 wt.% of vitamin E;

(II) providing a masterbatch comprising ≤ about 15 wt.%, preferably about 1 - 15 wt.% of zinc in the form of at least one zinc compound as described above pre-dispersed in a thermoplastic polymer, preferably polyethylene;

(III) mixing, in the molten state, the UHMWPE premixed with vitamin E with the masterbatch.

Step (I) can be carried out as described above. The masterbatch used in step (II) can be obtained by compounding, using twin-screw or single-screw extruders of the type normally used in the manufacture of thermoplastic polymers. The person skilled in the art will be able to determine, based on his technical knowledge, the process conditions (operating temperature and rotation speed) for obtaining a homogeneous dispersion of the at least one Zn compound in the thermoplastic polyolefin polymer.

In step (III), the UHMWPE doped with vitamin E can be mixed with the masterbatch by means of a ram extrusion process as described above, so as also to obtain, besides the mixing of the materials, the consolidation of the antibacterial polymeric material. The amount of masterbatch used in step (III) is such as to obtain a concentration ≤ 0.1 wt.% of Zn, preferably about 0.01-0.1 wt.%, more preferably about 0.03 - 0.09 wt.% of Zn in the antibacterial polymeric material obtained from the process.

The present invention further relates to an antibacterial polymeric material obtained or obtainable from the process as described above.

At the end of the consolidation step (iii), the consolidated antibacterial polymeric material obtained or obtainable from the above-described process can be used for the production of an orthopedic prosthetic device; alternatively, but less preferably, the antibacterial polymeric material can be granulated and subjected to further consolidation in order to produce an orthopedic prosthetic device.

The Applicant has surprisingly found that by doping UHMWPE with vitamin E and at least one organic zinc compound as specified above one obtains a material with high antibacterial activity associated with a low release of Zn, leaving the physical and mechanical characteristics of the polymer substantially unchanged.

Therefore, the antibacterial UHMWPE according to the present invention can have at least one of the following properties, preferably all of the following properties:

- crystallinity about 40-65%; and/or
- density $\geq$ about 0.930 g/cm$^3$, preferably comprised between about 0.930 and about 0.945 g/cm$^3$; and/or
- ultimate tensile strength $\geq$ about 35 MPa; and/or
- yield strength $\geq$ about 20 MPa; and/or
- elongation at break $\geq$ about 300%.

The antibacterial polymeric material according to the invention, obtained or obtainable from the above-described process, is therefore characterized by physical and mechanical properties that are optimal for its use in the production of an orthopedic prosthetic device. Preferably, the antibacterial polymeric material according to the invention can satisfy the requirements established by standard ASTM F2695-07.

Therefore, in a further aspect, the present invention relates to the use of the antibacterial polymeric material as described above for the production of an orthopedic prosthetic device, preferably for the production of at least one component of a total joint endoprosthesis.

According to a preferred variant, said at least one component can be the sliding surface of a joint endoprosthesis, for example the sliding surface of the hip, knee, shoulder, ankle, elbow or vertebral column.

In the present description and in the appended claims, the term "sliding surface" refers to an insert of a prosthetic joint device placed between the metal parts of the prosthesis, on which the joint moves (slides).

Figures 1 and 1A show a non-limiting example of a hip endoprosthesis, in which the sliding surface that can be produced using the antibacterial polymeric material of the invention is an acetabular cup (10).

In a further aspect, the present invention relates to an orthopedic prosthetic device comprising the antibacterial polymeric material as described above.

According to a first variant, said orthopedic prosthetic device antibacterial can be selected among total joint endoprostheses (arthroprostheses), preferably selected among endoprostheses of the hip, knee, shoulder, ankle, elbow and vertebral column.

In a further aspect thereof, the present invention relates to a sliding surface of a total joint endoprosthesis comprising the antibacterial polymeric material according to the invention, wherein said sliding surface is selected among an acetabular cup of a hip prosthesis, an acetabular cup of a shoulder prosthesis and an insert of a prosthesis of the knee (knee-joint component), ankle and elbow.

In a further aspect, the present invention relates to a process for the production of an orthopedic prosthetic device comprising the steps of:

(a) providing a consolidated antibacterial polymeric material according to the invention;
(b) cross-linking the antibacterial polymeric material; and
(b) molding the consolidated antibacterial UHMWPE.

The cross-linking can preferably take place by irradiation with high-energy ionizing radiation, even more preferably by irradiating with a total nominal dose of about 25-100 kGy, more preferably about 60-90 kGy, with beta and/or gamma rays.

Gamma rays are electromagnetic waves generated through the gamma decay of isotopes and they have a maximum penetration into parts of about 50 cm. Beta rays are accelerated electron beams that can penetrate into a part by a maximum of about 2.5 cm. In order to obtain a uniform irradiation on parts of a larger size, the parts are normally repeatedly irradiated by rotating them relative to the source of radiation. Preferably, when $\beta$ rays are used, the irradiation can be repeated until reaching the maximum nominal dose of 100 kGy.

The consolidated and irradiated antibacterial polymeric material is molded in step (c) using known techniques in order to obtain an antibacterial orthopedic prosthetic device of the desired geometry.

Optionally, upstream of step (a), the process can comprise a further heat stabilization step (O) at a temperature above 110°C. If the heat treatment is performed at a temperature below the melting temperature of the material (about 120°C) it is called annealing; if it is carried out at higher temperatures it is called remelting. The heat treatment has the purpose of further reducing the amount of free radicals present in the material due to the cross-linking treatment.

According to a further variant, the process can comprise a further sterilization step (d) downstream of step (c). The sterilization of the antibacterial orthopedic prosthetic device can be carried out with known techniques using ethylene oxide or plasma gas.

The present invention is illustrated below by means of the examples that are given in the experimental part, without limiting, however, the scope thereof.

**Analytical methods**

Thermal analysis:

*Method 1:* DSC 40°-400°C; method: UNI EN ISO 11357-1:2009 + ISO 11357-2:2013 + UNI EN ISO 11357-3:2013. Instruments: Heat-flux DSC with mass standard flow control Q20 - TA Instrument - Div. Waters SpA with a standard aluminum furnace for the samples and 5.0-50.0 ml/min of nitrogen as the purge gas. Two heating cycles and one cooling cycle.
*Method 2:* instrument: DSC Perkin Elmer Pyris 6. Heating from 40°C to 180°C at a rate of 10°C/min. The data refer to a mean of three measurements for each sample.

The crystallization percentage is determined by normalizing the melting temperature of the sample analyzed relative to the melting temperature of pure crystalline polyethylene (293 J/g).
Amount of zinc in the polymer: EPA 3050B:1996 + EPA 6010C:2007.
Small punch testing: ASTM F2183-02
Antibacterial kinetics: the antibacterial activity of test specimens of plastic material is evaluated using the following test bacteria

1. Staphylococcus epidermidis bacterium (Gram+) ATCC 12228*
2. Methicillin-resistant Staphylococcus aureus (MRSA) bacterium (Gram+) ATCC 25923*
3. Pseudomonas aeruginosa bacterium (Gram-) ATCC 9027*
4. Enterococcus faecalis bacterium (Gram+) ATCC 29212*
*ATCC (American Type Culture Collections).

Culture media:

Tryptone Soy Broth (TSBroth MERCK)
Tryptone Soy Agar (Caso Agar MERCK)
Diluent: saline solution
The method follows the guidelines of standard ISO 22196:2011. Preparation of the inoculum: the commercial bacterial suspensions were diluted with TSBroth so as to obtain a bacterial concentration expressed in colony-forming units (cfu)/ml.
The samples were inoculated with the reference microbial strains (0.4 ml - cfu/ml: $4.5 \times 10^4$ cfu/cm$^2$ of covered surface area), covered with a cover film, positioned in Petri dishes and placed in an incubator at a temperature of $37 \pm 1$°C for 24 hours, 48 hours, 5 days, 15 days and 20 days.
At the end of the incubation period, the samples under examination were washed with the neutralizing agent (peptone and lecithin solution at pH=7) in order to determine the residual viable microbial load (microscopic count of CFUs on the contact plate).
The result of the reference test specimen after each contact time (Ut $N_{24}$ hours, Ut $N_{48}$ hours, Ut $N_5$ days, Ut $N1_5$ days and Ut $N_{20}$ days) was compared with the respective result obtained with the test samples subjected to each contact time.
Every test was performed in triplicate.

**Examples 1-5 and comparative Example 6**

The samples of antibacterial polymeric material used in examples 1-5 were prepared by mixing (ram extrusion) GUR Celanese 1050E, a UHMWPE doped with 0.1 wt.% of vitamin E sold by Celanese Corp., with different percentages of a masterbatch based on polyethylene containing zinc acetate.
The data regarding the comparative example 6 were acquired from a commercial sample of UHMWPE doped with vitamin E as such, without the zinc compound.
The results of the thermal analyses performed to characterize the samples according to *Method 1* are shown in Table 1.

**Table 1**

|  | Ex. 1 | Ex. 2 | Ex. 3 | Ex. 4 | Ex.5 | Comp. Ex. 6 |
|---|---|---|---|---|---|---|
| **Melting temperature (°C)** |  |  |  |  |  |  |
| $T_{mp}$ (peak) | 131 | 130 | 130 | 131 | 130 | 130 |
| $T_{eim}$ (onset) | 121 | 122 | 121 | 122 | 121 | 121 |
| $T_{efm}$ (end) | 131 | 135 | 135 | 136 | 135 | 134 |
| **Crystallisation temperature (°C)** |  |  |  |  |  |  |
| $T_{cp}$ (peak) | 115 | 115 | 115 | 114 | 115 | 115 |
| $T_{eic}$ (onset) | 118 | 118 | 118 | 118 | 118 | 116 |
| $T_{efc}$ (end) | 111 | 111 | 111 | 110 | 111 | 112 |
| Zinc (mg/Kg) | 876 | 755 | 421 | 250 | 156 | / |

**Examples 7-11 and comparative example 12**

Plates having dimensions of 50x50x10 mm were obtained from the samples prepared in examples 1-5 and in the comparative example 6. The plates were cross-linked by irradiation with β rays at a nominal dose of 25 kGy and 100 kGy. Test specimens of a circular shape (diameter 6.4 mm), 0.5 mm thick, were subsequently obtained from the plates of consolidated and irradiated antibacterial polymeric material.

The samples were subjected to small punch testing at a speed of 0.5 mm/min at room temperature. Each measurement was repeated three times; Table 2a shows the mean values of the three measurements for the samples irradiated with a nominal dose of 25 kGy; Table 2b shows the mean values of the measurements performed on samples cross-linked with a nominal dose of 100 kGy.

**Table 2a - nominal radiation dose 25 kGy**

| EX. | Zn. Conc. (mg/Kg) | Initial peak load (N) | Ultimate load (N) | Elongation at break (mm) |
|---|---|---|---|---|
| 7 | 876 | 67.6 | 67.0 | 3.6 |
| 8 | 755 | 60.0 | 64.9 | 3.8 |
| 9 | 421 | 59.6 | 64.3 | 4.2 |
| 10 | 250 | 63.0 | 63.6 | 3.8 |
| 11 | 156 | 57.1 | 68.4 | 4.5 |
| Comp. 12 | / | 60.6 | 72.1 | 4.8 |

**Table 2b - nominal radiation dose 100 kGy**

| EX. | Zn. Conc. (mg/Kg) | Initial peak load (N) | Ultimate load (N) | Elongation at break (mm) |
|---|---|---|---|---|
| 7 | 876 | 62.0 | 65.3 | 3.0 |
| 8 | 755 | 68.0 | 78.3 | 3.5 |
| 9 | 421 | 68.8 | 77.1 | 3.7 |
| 10 | 250 | 66.3 | 78.4 | 3.9 |
| 11 | 156 | 63.8 | 86.1 | 4.6 |
| Comp. 12 | / | 65.9 | 82.0 | 4.3 |

Both the initial peak load and the ultimate load increase with increases in the nominal radiation dose. The variation in mechanical properties, in terms of ultimate load and elongation at break, is very small and increases as the amount of zinc added increases. However, it shows to be of an entity such as not to compromise the use of the material in the prosthetics field.

Samples weighing approximately 4 mg of the samples of consolidated and irradiated antibacterial polymeric material prepared as described above were subjected to DSC according to *Method 2* to determine the melting temperature ($T_{pf}$) and percent crystallinity.

Table 3a shows the values measured in samples irradiated with a nominal dose of 25 kGy; Table 3b the values measured in samples irradiated with a nominal dose of 25 kGy.

### Table 3a - nominal radiation dose 25 kGy

| EX. | Zn. Conc. (mg/Kg) | Melting Temp. $T_{mp}$ (°C) | Crystallinity (%) |
|---|---|---|---|
| 7 | 876 | 138.8 | 47.7 |
| 8 | 755 | 138.8 | 46.1 |
| 9 | 421 | 138.2 | 46.4 |
| 10 | 250 | 138.1 | 44.4 |
| 11 | 156 | 138.0 | 45.4 |
| Comp. 12 | / | 137.6 | 46.0 |

### Table 3b - nominal radiation dose 100 kGy

| EX. | Zn. Conc. (mg/Kg) | Melting Temp. $T_{mp}$ (°C) | Crystallinity (%) |
|---|---|---|---|
| 7 | 876 | 140.7 | 49.1 |
| 8 | 755 | 140.7 | 49.1 |
| 9 | 421 | 140.4 | 47.4 |
| 10 | 250 | 139.1 | 48.4 |
| 11 | 156 | 140.0 | 44.2 |
| Comp. 12 | / | 139.8 | 47.4 |

As the radiation dose increases, a slight increase in crystallinity can be observed. No significant difference in crystallinity was observed among the various samples.

### Examples 13-17

The 50x50x100 mm plates of consolidated and irradiated antibacterial polymeric material obtained in examples 7-11 were subjected to an antibacterial kinetics test to evaluate antimicrobial effectiveness.

A 50x50x100 mm test specimen obtained from GUR Celanese 1050E, sold by Celanese Corp., was used as a reference (Ut).

The following Tables 4a-d show the experimental results for the different microbial strains.

### Table 4a - *Staphylococcus epidermidis* ATCC 12228

| | | Ex. 13 | Ex. 14 | Ex. 15 | Ex. 16 | Es.17 | Rif. |
|---|---|---|---|---|---|---|---|
| Zinc (mg/Kg) | | 876 | 755 | 421 | 250 | 156 | / |
| 24 h | Ut | / | / | / | / | / | 3.00E+05 |
| | At | 1.00E+04 | 1.80E+04 | 2.00E+04 | 2.40E+04 | 2.80E+04 | |
| | R log | 1.74 | 1.51 | 1.20 | 1.20 | 1.09 | |
| | Red % | 96.7 | 94.0 | 93.3 | 92.0 | 90.7 | |

(continued)

|  |  | Ex. 13 | Ex. 14 | Ex. 15 | Ex. 16 | Es.17 | Rif. |
|---|---|---|---|---|---|---|---|
| 48 h | Ut | / | / | / | / | / | 2.60E+05 |
| | At | 2.00E+02 | 3.00E+02 | 3.90E+03 | 9.00E+03 | 1.00E+04 | |
| | R log | 1.14 | 1.02 | 0.91 | 0.86 | 0.66 | |
| | Red % | 99.9 | 99.9 | 98.5 | 96.5 | 96.2 | |
| 5d | Ut | / | / | / | / | / | 6.00E+04 |
| | At | 9.00E+00 | 9.00E+00 | 1.00E+02 | 1.00E+03 | 1.20E+03 | |
| | R log | 1.14 | 1.02 | 0.91 | 0.86 | 0.66 | |
| | Red % | 99.99 | 99.99 | 99.8 | 98.3 | 98.0 | |
| 15d | Ut | / | / | / | / | / | 1.80E+04 |
| | At | 9.00E+00 | 9.00E+00 | 9.00E+00 | 5.00E+02 | 8.00E+02 | |
| | R log | 1.14 | 1.02 | 0.91 | 0.86 | 0.66 | |
| | Red % | 99.95 | 99.95 | 99.95 | 97.2 | 95.6 | |
| 20d | Ut | / | / | / | / | / | 3.40E+03 |
| | At | 9.00E+00 | 9.00E+00 | 9.00E+00 | 5.00E+02 | 5.00E+02 | |
| | R log | 1.14 | 1.02 | 0.91 | 0.86 | 0.66 | |
| | Red % | 99.74 | 99.74 | 99.74 | 85.3 | 85.3 | |

Ut = bacterial count in the reference after time t
At = mean of the logarithm of the bacterial count after time t
R log = antibacterial activity (Ut - At) expressed as a logarithm

$$Red\% = (final\ load - initial\ load)/(initial\ load) \times 100$$

**Table 4b - Staphylococcus aureus (MRSA) ATCC 25923**

|  |  | Ex. 13 | Ex. 14 | Ex. 15 | Ex. 16 | Es.17 | Rif. |
|---|---|---|---|---|---|---|---|
| Zinc (mg/Kg) | | 876 | 755 | 421 | 250 | 156 | / |
| 24 h | Ut | / | / | / | / | / | 2.60E+05 |
| | At | 1.00E+04 | 2.0E+04 | 2.50E+04 | 2.80E+04 | 3.0E+04 | |
| | R log | 1.74 | 1.51 | 1.20 | 1.20 | 1.09 | |
| | Red % | 96.2 | 92.3 | 90.4 | 89.2 | 88.5 | |
| 48 h | Ut | / | / | / | / | / | 2.40E+05 |
| | At | 1.00E+03 | 1.20E+03 | 2.00E+03 | 1.00E+04 | 1.20E+04 | |
| | R log | 1.14 | 1.02 | 0.91 | 0.86 | 0.66 | |
| | Red % | 99.6 | 99.5 | 99.2 | 95.8 | 95.0 | |
| 5d | Ut | / | / | / | / | / | 2.00E+04 |
| | At | 9.00E+00 | 9.00E+00 | 9.00E+00 | 1.00E+03 | 1.30E+03 | |
| | R log | 1.14 | 1.02 | 0.91 | 0.86 | 0.66 | |
| | Red % | 99.96 | 99.96 | 99.96 | 95.0 | 93.5 | |

(continued)

|  |  | Ex. 13 | Ex. 14 | Ex. 15 | Ex. 16 | Es.17 | Rif. |
|---|---|---|---|---|---|---|---|
| 15d | Ut | / | / | / | / | / | 1.60E+04 |
|  | At | 9.00E+00 | 9.00E+00 | 9.00E+00 | 4.00E+02 | 1.00E+03 |  |
|  | R log | 1.14 | 1.02 | 0.91 | 0.86 | 0.66 |  |
|  | Red % | 99.94 | 99.94 | 99.94 | 97.5 | 93.8 |  |
| 20d | Ut | / | / | / | / | / | 6.40E+03 |
|  | At | 9.00E+00 | 9.00E+00 | 9.00E+00 | 3.00E+02 | 1.00E+02 |  |
|  | R log | 1.14 | 1.02 | 0.91 | 0.86 | 0.66 |  |
|  | Red % | 99.86 | 99.86 | 99.86 | 95.3 | 84.4 |  |

### Table 4c - *Pseudomonas aruginosa ATCC 9027*

|  |  | Ex. 13 | Ex. 14 | Ex. 15 | Ex. 16 | Es.17 | Rif. |
|---|---|---|---|---|---|---|---|
| Zinc (mg/Kg) |  | 876 | 755 | 421 | 250 | 156 | / |
| 24 h | Ut | / | / | / | / | / | 2.00E+05 |
|  | At | 9.00E+03 | 1.00E+04 | 1.00E+04 | 1.60E+04 | 2.00E+04 |  |
|  | R log | 1.74 | 1.51 | 1.20 | 1.20 | 1.09 |  |
|  | Red % | 95.5 | 95.0 | 95.0 | 92.0 | 90.0 |  |
| 48 h | Ut | / | / | / | / | / | 1.90E+05 |
|  | At | 5.00E+02 | 1.00E+03 | 2.00E+03 | 3.00E+03 | 3.00E+03 |  |
|  | R log | 1.14 | 1.02 | 0.91 | 0.86 | 0.66 |  |
|  | Red % | 99.7 | 99.5 | 98.9 | 98.4 | 98.4 |  |
| 5d | Ut | / | / | / | / | / | 1.60E+05 |
|  | At | 9.00E+00 | 9.00E+00 | 9.00E+00 | 1.00E+03 | 1.50E+03 |  |
|  | R log | 1.14 | 1.02 | 0.91 | 0.86 | 0.66 |  |
|  | Red % | 99.994 | 99.994 | 99.994 | 99.4 | 99.1 |  |
| 15d | Ut | / | / | / | / | / | 2.80E+04 |
|  | At | 9.00E+00 | 9.00E+00 | 9.00E+00 | 1.00E+03 | 1.00E+03 |  |
|  | R log | 1.14 | 1.02 | 0.91 | 0.86 | 0.66 |  |
|  | Red % | 99.968 | 99.968 | 99.968 | 96.4 | 96.4 |  |
| 20d | Ut | / | / | / | / | / | 4.00E+03 |
|  | At | 9.00E+00 | 9.00E+00 | 9.00E+00 | 1.00E+03 | 1.00E+03 |  |
|  | R log | 1.14 | 1.02 | 0.91 | 0.86 | 0.66 |  |
|  | Red % | 99.78 | 99.78 | 99.78 | 75.0 | 75.0 |  |

### Table 4d - *Enterococcus faecalis ATCC 29212*

|  | Ex. 13 | Ex. 14 | Ex. 15 | Ex. 16 | Es.17 | Rif. |
|---|---|---|---|---|---|---|
| Zinc (mg/Kg) | 876 | 755 | 421 | 250 | 156 | / |

(continued)

|  |  | Ex. 13 | Ex. 14 | Ex. 15 | Ex. 16 | Es.17 | Rif. |
|---|---|---|---|---|---|---|---|
| 24 h | Ut | / | / | / | / | / | 1.90E+05 |
|  | At | 7.00E+03 | 1.00E+04 | 2.00E+04 | 2.00E+04 | 2.30E+04 |  |
|  | R log | 1.74 | 1.51 | 1.20 | 1.20 | 1.09 |  |
|  | Red % | 96.3 | 94.7 | 89.5 | 89.5 | 87.9 |  |
| 48 h | Ut | / | / | / | / | / | 1.00E+05 |
|  | At | 1.90E+02 | 3.00E+02 | 1.00E+04 | 1.00E+04 | 1.80E+04 |  |
|  | R log | 1.14 | 1.02 | 0.91 | 0.86 | 0.66 |  |
|  | Red % | 99.8 | 99.7 | 90.0 | 90.0 | 82.0 |  |
| 5d | Ut | / | / | / | / | / | 4.50E+04 |
|  | At | 9.00E+00 | 9.00E+00 | 9.00E+00 | 1.00E+03 | 1.50E+03 |  |
|  | R log | 1.14 | 1.02 | 0.91 | 0.86 | 0.66 |  |
|  | Red % | 99.98 | 99.98 | 99.33 | 97.78 | 97.33 |  |
| 15d | Ut | / | / | / | / | / | 1.00E+04 |
|  | At | 9.00E+00 | 9.00E+00 | 1.00E+03 | 1.00E+03 | 1.00E+03 |  |
|  | R log | 1.14 | 1.02 | 0.91 | 0.86 | 0.66 |  |
|  | Red % | 99.91 | 99.91 | 99.00 | 90.00 | 90.00 |  |
| 20d | Ut | / | / | / | / | / | 4.00E+03 |
|  | At | 9.00E+00 | 9.00E+00 | 9.00E+00 | 9.00E+02 | 1.00E+03 |  |
|  | R log | 1.14 | 1.02 | 0.91 | 0.86 | 0.66 |  |
|  | Red % | 99.74 | 99.74 | 99.74 | 73.5 | 70.6 |  |

The tests performed show a progressive reduction in the bacterial load for all of the strains tested, both directly in proportion to the zinc concentration and with increases in the time of contact with the various bacterial strains.

**Claims**

1. An antibacterial polymeric material comprising ultrahigh molecular weight polyethylene, $\leq 0,3$ wt.% of vitamin E and $\leq 0,1$ w.t% of Zn, wherein Zn is added to the ultrahigh molecular weight polyethylene in the form of a zinc compound selected among an inorganic zinc salt, a zinc carboxylate, zinc pyrithione, zinc acetylacetonate and mixtures thereof.

2. The antibacterial polymeric material according to claim 1, comprising 0,05 - 0,3 wt.%, preferably 0,1 wt.% of vitamin E.

3. The antibacterial polymeric material according to claim 1 or 2, comprising 0,01-0,1 wt.% of Zn, preferably 0,03 - 0,09 wt.%.

4. The antibacterial polymeric material according to any one of claims from 1 to 3, wherein the at least one zinc compound is selected among zinc dichloride, zinc sulfate, zinc carbonate, zinc basic carbonate, zinc nitrate, zinc phosphate and mixtures thereof, preferably zinc carbonate.

5. The antibacterial polymeric material according to any one of claims from 1 to 3, wherein the at least one zinc compound is selected among zinc compounds with carboxylic acids of formula $R^1C(=O)OH$ and/or the anions of said carboxylic acids, wherein $R^1$ is selected among C1-C17 alkyl, C1-C10 substituted alkyl, C1-C10 alkenyl, phenyl, substituted phenyl, heterophenyl and pyrrolidone.

**EP 3 290 060 B1**

6. The antibacterial polymeric material according to claim 5, wherein the at least one zinc compound is selected among zinc acetate, zinc propionate, zinc acrylate, zinc methacrylate, zinc oxalate, zinc aspartate, zinc citrate, zinc gluconate, zinc neodecanoate, zinc undecylenate, zinc stearate, zinc PCA, zinc picolinate, zinc salicylate and mixtures thereof, preferably among zinc acetate and zinc stearate.

7. A process for the preparation of the antibacterial polymeric material according to any one of claims from 1 to 6, comprising mixing UHMWPE with ≤ 0,3 wt.% of vitamin E and ≤ 0,1 wt.% of Zn, in the form of at least one zinc compound selected among an inorganic zinc salt, a zinc carboxylate, zinc pyrithione, zinc acetylacetonate and mixtures thereof, wherein the mixing is carried out in the dry state, in solution or in the molten state.

8. Use of the antibacterial polymeric material according to any one of claims from 1 to 6 for the production of an orthopedic prosthetic device, preferably for the production of at least one component of a total endoprosthetic joint.

9. An orthopedic prosthetic device comprising the antibacterial polymeric material according to any one of claims from 1 to 6.

10. The orthopedic prosthetic device according to claim 9, wherein said device is a total endoprosthetic joint, preferably selected among an endoprosthesis of the hip, knee, shoulder, ankle, elbow and vertebral column.

**Patentansprüche**

1. Antibakterielles polymerisches Material, umfassend Polyethylen mit ultrahohem Molekulargewicht, ≤ 0,3 Gew.-% Vitamin E und ≤ 0,1 Gew.-% Zn, wobei Zn dem Polyethylen mit ultrahohem Molekulargewicht in Form einer Zinkverbindung zugesetzt ist, die unter einem anorganischen Zinksalz, einem Zinkcarboxylat, Zinkpyrithion, Zinkacetylacetonat und Mischungen davon ausgewählt ist.

2. Antibakterielles polymerisches Material nach Anspruch 1, umfassend 0,05-0,3 Gew.-%, vorzugsweise 0,1 Gew.-% Vitamin E.

3. Antibakterielles polymerisches Material nach Anspruch 1 oder 2, umfassend 0,01-0,1 Gew.-% Zn, vorzugsweise 0,03-0,09 Gew.-%.

4. Antibakterielles polymerisches Material nach einem der Ansprüche 1 bis 3, wobei die mindestens eine Zinkverbindung unter Zinkdichlorid, Zinksulfat, Zinkcarbonat, Zinkbasiscarbonat, Zinknitrat, Zinkphosphat und Mischungen davon, vorzugsweise Zinkcarbonat ausgewählt ist.

5. Antibakterielles polymerisches Material nach einem der Ansprüche 1 bis 3, wobei die mindestens eine Zinkverbindung unter Zinkverbindungen mit Carbonsäuren der Formel $R^1C(=O)OH$ und/oder den Anionen der Carbonsäuren ausgewählt ist, wobei $R^1$ aus C1-C17-Alkyl, C1-C10-substituiertem Alkyl, C1-C10-Alkenyl, Phenyl, substituiertem Phenyl, Heterophenyl und Pyrrolidon ausgewählt ist.

6. Antibakterielles polymerisches Material nach Anspruch 5, wobei die mindestens eine Zinkverbindung unter Zinkacetat, Zinkpropionat, Zinkacrylat, Zinkmethacrylat, Zinkoxalat, Zinkaspartat, Zinkcitrat, Zinkgluconat, Zinkneodecanoat, Zinkundecylenat, Zinkstearat, Zink-PCA, Zink-Picolinat, Zinksalicylat und Mischungen davon, vorzugsweise unter Zinkacetat und Zinkstearat ausgewählt ist.

7. Verfahren zur Herstellung des antibakteriellen polymerischen Materials nach einem der Ansprüche 1 bis 6, umfassend Mischen von UHMWPE mit ≤ 0,3 Gew.-% Vitamin E und ≤ 0,1 Gew.-% Zn in Form von mindestens einer Zinkverbindung, ausgewählt unter einem anorganischen Zinksalz, einem Zinkcarboxylat, Zinkpyrithion, Zinkacetylacetonat und Mischungen davon, wobei das Mischen im trockenen Zustand, in Lösung oder im geschmolzenen Zustand durchgeführt wird.

8. Verwendung des antibakteriellen polymerischen Materials nach einem der Ansprüche 1 bis 6 zur Herstellung einer orthopädischen Prothesenvorrichtung, vorzugsweise zur Herstellung mindestens einer Komponente eines totalendoprothetischen Gelenks.

9. Orthopädische Prothesenvorrichtung, umfassend das antibakterielle polymerische Material nach einem der Ansprüche 1 bis 6.

**10.** Orthopädische Prothesenvorrichtung nach Anspruch 9, wobei die Vorrichtung ein totalendoprothetisches Gelenk ist, vorzugsweise ausgewählt unter einer Endoprothese der Hüfte, des Knies, der Schulter, des Fußgelenks, des Ellbogens und der Wirbelsäule.

## Revendications

**1.** Matériau polymère antibactérien comprenant du polyéthylène de poids moléculaire ultra élevé, $\leq 0,3$ % en poids de vitamine E et $\leq 0,1$ % en poids de Zn, dans lequel Zn est ajouté au polyéthylène de poids moléculaire ultra élevé sous forme d'un composé de zinc sélectionné parmi un sel de zinc inorganique, un carboxylate de zinc, un pyrithione de zinc, un acétylacétonate de zinc et leurs mélanges.

**2.** Matériau polymère antibactérien selon la revendication 1, comprenant 0,05 - 0,3 % en poids, de préférence 0,1 % en poids de vitamine E.

**3.** Matériau polymère antibactérien selon la revendication 1 ou 2, comprenant 0,01 - 0,1 % en poids, de préférence 0,03 - 0,09 % en poids de Zn.

**4.** Matériau polymère antibactérien selon l'une quelconque des revendications de 1 à 3, dans lequel l'au moins un composé de zinc est sélectionné parmi le dichlorure de zinc, le sulfate de zinc, le carbonate de zinc, le carbonate basique de zinc, le nitrate de zinc, le phosphate de zinc et leurs mélanges, de préférence le carbonate de zinc.

**5.** Matériau polymère antibactérien selon l'une quelconque des revendications de 1 à 3, dans lequel l'au moins un composé de zinc est sélectionné parmi des composés de zinc comportant des acides carboxyliques de formule $R^1C(=O)OH$ et/ou des anions desdits acides carboxyliques, dans lequel $R^1$ est sélectionné parmi un alkyle en C1-C17, un alkyle substitué en C1-C10, un alcényle en C1-C10, un phényle, un phényl substitué, un hétérophényle et un pyrrolidone.

**6.** Matériau polymère antibactérien selon la revendication 5, dans lequel l'au moins un composé de zinc est sélectionné parmi l'acétate de zinc, le propionate de zinc, l'acrylate de zinc, le méthacrylate de zinc, l'oxalate de zinc, l'aspartate de zinc, le citrate de zinc, le gluconate de zinc, le néodécanoate de zinc, le undecylenate de zinc, le stéarate de zinc, le zinc PCA, le picolinate de zinc, le salicylate de zinc et leurs mélanges, de préférence parmi l'acétate de zinc et le stéarate de zinc.

**7.** Procédé de préparation d'un matériau polymère antibactérien selon l'une quelconque des revendications de 1 à 6, comprenant le mélange de l'UHMWPE avec $\leq 0,3$ % en poids de vitamine E et $\leq 0,1$ % en poids de Zn, sous forme d'au moins un composé de zinc sélectionné parmi un sel de zinc inorganique, un carboxylate de zinc, un pyrithione de zinc, un acétylacétonate de zinc et leurs mélanges, dans lequel le mélange est réalisé à l'état sec, en solution ou à l'état fondu.

**8.** Utilisation d'un matériau polymère antibactérien selon l'une quelconque des revendications de 1 à 6 pour la fabrication d'une prothèse orthopédique, de préférence pour la fabrication d'au moins un composant d'une articulation endoprothétique totale.

**9.** Prothèse orthopédique comprenant le matériau polymère antibactérien selon l'une quelconque des revendications de 1 à 6.

**10.** Prothèse orthopédique selon la revendication 9, dans laquelle ladite prothèse est une articulation endoprothétique totale, de préférence sélectionnée parmi une endoprothèse de la hanche, du genou, de l'épaule, de la cheville, du coude et de la colonne vertébrale.

FIG. 1

FIG. 1A

10

10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014024736 A1

**Non-patent literature cited in the description**

- **F. CIUCIO et al.** *Bollettino of the società medico chirurgica of Pavia,* 2013, vol. 126 (2), 421-430